(19) Europäisches Patentamt
European Patent Office
Office européen des brevets

(11) Publication number: **0 025 576**
**B1**

(12) **EUROPEAN PATENT SPECIFICATION**

(45) Date of publication of patent specification: **14.01.87**

(51) Int. Cl.$^4$: **G 01 F 1/36**, G 01 F 1/72, A 61 B 5/00

(21) Application number: **80105351.3**

(22) Date of filing: **08.09.80**

# (54) Turbulence conveyor flowmeter for medical use.

(30) Priority: **14.09.79 IT 8413879**

(43) Date of publication of application:
**25.03.81 Bulletin 81/12**

(45) Publication of the grant of the patent:
**14.01.87 Bulletin 87/03**

(84) Designated Contracting States:
**AT BE CH DE FR GB LI LU NL SE**

(56) References cited:
**DE-B-1 295 254**
**GB-A-2 026 704**
**US-A-2 606 445**
**US-A-2 675 020**
**US-A-3 294 361**
**US-A-3 817 099**

(73) Proprietor: **Torresin, Giuseppe**
**Via Praarie, 28,**
**I-35010 San Giorgio Delle Pertiche (Padova) (IT)**

(73) Proprietor: **Succu, Francesca**
**Via Praarie, 28**
**I-35010 San Giorgio Delle Pertiche (Padova) (IT)**

(72) Inventor: **Torresin, Giuseppe**
**Via Praarie, 28**
**I-35010 San Giorgio Delle Pertiche (IT)**

(74) Representative: **Görtz, Dr. Fuchs, Dr.**
**Luderschmidt Patentanwälte**
**Sonnenberger Strasse 100 Postfach 26 26**
**D-6200 Wiesbaden (DE)**

**EP 0 025 576 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention concerns a turbulence conveyor gas flowmeter for medical use, according to the preamble part of claim 1.

A generic flowmeter is for example known from U.S. Patent US—A—2,606,445. This known device comprises a body portion including a cavity having at least two openings for input and/or output of gasflow. Furthermore, there is provided a movable intercepting element within said cavity with at least a turbulence chamber being adapted to get into connection with said openings and having input and/or output openings of variable sectional area due to the movement of said intercepting element with a gas flowing from one input to one output through the chamber. To respond to the variable pressure drops across the openings there is provided a transducer system which is connected to the openings by means of ducts. A servomechanism controlled by said transducer system moves said intercepting element in form of a shuttle or valve member which due to its geometry closes a throughport to varying degrees.

The main disadvantage of this known flowmeter, however, is that it is not adapted to be used in the medical field as particularly for monitoring human respiratory functions. The reason for this is that the known flowmeter is adapted to respond to a constant flow system. The use of flowmeters in the medical field as mentioned above, however, requires the possibility of adjusting the flow as a function of a compliant system such as a patient's lungs.

It is, therefore, the problem underlying the present invention to provide a turbulence conveyor gas flowmeter according to the preamble part of claim 1 that is able to respond to the flow as a function of a compliant system thereby making it possible to use the flowmeter particularly for monitoring human respiratory functions.

This problem is solved by the characterizing features of claim 1.

Although from U.S. Patent US—A—2,675,020 a flowmeter with variable orifice is known using pressure difference for control purposes, there is no indication of the provision of a body portion with three openings and of computing means as with the flowmeter according to the invention.

The subclaims comprise advantageous embodiments of the present invention.

The present invention will be further discussed hereinafter in connection with some preferred embodiments, given as non-limitative examples, with reference to the accompanying sheets of drawings, wherein:

Fig. 1 is a partial sectional perspective view of a flowmeter useful to illustrate the invention, comprising a one-way tab as intercepting element;

Fig. 2 is a sectional view of the flowmeter of Fig. 1;

Fig. 3a is a sectional view of the flowmeter according to the invention provided with a two-ways tab;

Figs. 3b and 3c are sectional views of two-ways tabs of Fig. 3a, in different operational conditions,

Fig. 4 is a sectional view of a flowmeter according to the invention, with one pressure transducer;

Fig. 5 is a sectional view of a different embodiment of the tab of the flowmeter according to the invention, and

Fig. 6 is a sectional view of a further embodiment of the tab of the flowmeter.

Fig. 1 and 2 represent a flowmeter useful to illustrate the invention and which includes a tap 1, formed by a body 2 out of which a spherical cavity has been grooved, and a spherical intercepting element 3, troused in said cavity. Out of the intercepting element 3, a diametral duct 4 has been grooved, which for a given position of the intercepting element 3, with respect to the body 2, is axially in line with two passage ways 5, 5′, grooved out of said body 2. Said passage ways 5, 5′ extend beyond the body 2 into two external conduits 6, 6′, whereof the conduit 6 is in contact with the patient's mouth, whereas the conduit 6′ is open to external air. In the interception element 3 moreover two coaxial ducts 7, 7′ are grooved. The axis of the coaxial pipes 7, 7′ is orthogonal to the axis of the duct 4 and coincides with the rotation axis of element 3 with respect to body 2. The two ducts 7, 7′ extend into the body 2 of tap 1 in further ducts 9, 9′, and then outwards into conduits 10, 10′.

From the ducts 6, 6′ start respectively conduits 11, 11′, communicating with a pressure transducer 12, which transforms the difference of pressure Δp observed into electrical signals. These signals are sent throught the connecting element 13 to a servomechanism 14, which controls the angular position of the intercepting element 3 around the rotation axis 8. The signal generated by the pressure transducer 12 is also sent, through a connection 15 to a microprocessor 16. Through a further connecting element 17, an electrical signal related to the angular position of the intercepting element 3 is sent from the servomechanism 14 to the microprocessor 16. A further connecting element 30 connects the microprocessor 16 to the servomechanism 14 to control the latter, as it will be further explained hereinafter.

Said flowmeter operates as follows:

The duct 4 of the intercepting element 3 is the turbulence chamber, into which the flow enters, through the passage way 5 (inspiration) or 5′ (expiration).

When no pressure signal is present (flow zero between inspiration and expiration) the transducer 12 acts on the servomechanism 14 in such a way that the latter keeps closed the tap 1. The starting of the expiration or inspiration phase causes a pressure increase in the conduit 6, with respect to conduit 6′, and when such an overpressure exceeeds a given threshold, the corresponding electric signal generated by the transducer 12 acts on the servomechanism 14 which opens the tap 1.

If the pressure transducer 12 gives a reliable response only within a very limited pressure range, it will be preferable that it acts on the servomechanism in such a way that the position of the intercepting element 3 of tap keeps a constant pressure drop in conduits 6, 6', during the whole expiration phase. The angular position of the intercepting element 3, transformed in an electrical signal within the same servomechanism 14, is sent to the microprocessor 16. The latter, keeping into account the value of $\Delta p$, coming from the transducer 12, gives the legible value of flow Q. The signal processing of the microprocessor 16 is in general of the type.

$$Q=f(A_1, A_2) \cdot (\Delta p)^{1/2} \qquad (1)$$

wherein $A_1$, $A_2$ are the areas of the input and output openings of the turbulence chamber 4. In the given example, of Figs. 1 and 2 if A denotes the common value of $A_1$ and $A_2$, the formula (1) can be written in the following way:

$$Q=G(A) \cdot A \cdot (\Delta p)^{1/2} \qquad (2)$$

wherein g(A) represents a suitable coefficient, which, for a given geometrical structure, can be held as a constant, thus simplifying the measurement. The use of the microprocessor 16, however, allows the calculation of expressions of type (1) in whole generality.

Moreover the microprocessor 16 allows to act on servomechanism 14, also in a wider case of the simplified one, above referred to i.e. in the case in which, during the whole expiration or inspiration phase, the difference of pressure $\Delta p$, in the conduits 6, 6', is not kept constant. The choice of the control policy of the angular displacement of the intercepting element 3, as function of $\Delta p$, depends upon the use of the conveyor flowmeter, its gemometry and upon the feature of the transducer.

If during the operation of the conveyor flowmeter a continuous collection of samples of the breathed flows has to be carried out, this can be done through duct 7. At the same time, to avoid any interference in the measurement, an equal volume of air or gas is sent into the duct 7'.

In the embodiment illustrated in Fig. 3a, 3b and 3c, the intercepting element 18 of the tap is provided with several ways, connecting, according to the angular position of said intercepting element, the conduit 19, at the patient's mouth, with the input conduit 20 or with the separate output 21 conduit.

Two pressure transducers 22 and 23 control the pressure variation, between the conduit 19 and conduits 20 and 21, respectively, and transform such variations into electrical signals, which are sent to a microprocessor 24 and to a servomechanism 25. The latter controls the angular position of the intercepting element 18 of the tap, whereas the microprocessor gives the flow signal Q.

In such an embodiment the conveyor flowmeter according to the invention operates as follows:

When there is no pressure signal, the tap remains closed (Fig. 3a). When the expiration phase starts, there is an increase of pressure in conduit 19, with respect to conduits 20 and 21. The two pressure variations, transformed into electrical signals by the two transducers 22 and 23, are sent to the microprocessor 24, which, acting on servo-mechanism 25, puts into communication the conduits 19 and 21 (cf. Fig. 3b). When the flowmeter has such a configuration, it operates as the flowmeter described above.

At the end of the expiration phase, the signal $\Delta p=0$ brings the intercepting element 18 back to the closed position (cf. Fig. 3a).

When the inspiration phase begins, there is a lowering of pressure in the conduit 19 with respect to the conduits 20 and 21. The two pressure variations, transformed into electrical signals by the two transducers 22 and 23, are sent to the microprocessor 24, which acting on the servo-mechanism 25 puts into communication the two conduits 19 and 20 (cf. Fig. 3c).

In Fig. 3a, to simplify things, two pressure transducers 22 and 23, have been illustrated. It is possible, however, to use only one pressure transducer 26 (cf. Fig. 4); when $\Delta p=0$ and the intercepting element is closed, such a transducer 26 communicates with both ducts 20 and 21 through valves 27 and 28, each of which is closed due to the action of mircoprocessor 24, when $\Delta p>0$ or $\Delta p<0$, respectively.

In a different embodiment (not shown in the drawings) the flowmeter has more than one way and an intercepting element of the one-way type. Even if this embodiment increases the dead space of the flowmeter, it allows to reduce the size of the intercepting element.

In the embodiment illustrated in Fig. 5 the tap of the flowmeter is of the three-ways, type. In this a case the intercepting element 29 is built in such a way that the opening connected to the patient is always open, for whichever angular position of the intercepting element.

In the embodiment of Fig. 6, the tap of the flowmeter is of the piston type, particularly useful when the servo-mechanism acts according to an axial movement.

From what has been said and independently from the particular embodiment, it clearly appears that the conveyor flowmeter according to the invention, offers several advantages, among which the following ones present a special interest:

—the possibility of measuring flows within a wide range of values, to answer all the requirements in the medical field. This possibility is due to the presence of the turbulence chamber and to the servo-adjustment of the area of the input and/or output openings;

—the practical insensitivity to dirtiness, as the turbulence is not obtained by means of laminas or obstacles of any other type, but only with simple holes;

—the facility of cleaning and sterilizing;

—the possibility, thanks to the use of microprocessor, of using transducers of every kind of response, including the non linear ones, so far as they are reliable, and therefore the possibility of manufacturing low-cost equipments;

—a remarkable reduction of the dead space, with respect to the flowmeters coupled to a separate conveyor, currently used, and therefore the possibility of carrying out measurement and tests in the medical field, practically without limitations;

—the possibility of carrying out flow measurements during rebreathing tests under the most variable conditions (stress testing, maximum forced expiration, etc.) due to the coupling in a unique device of the flow measuring and the conveying, within a wide range of values, and therefore the possibility of carrying-out from now on high-level researches and measurements in the physiological and clinical field;

—the possibility to perform with the same unique instrument the evaluation of the resistance of the airways with the flow interruption method;

—the possibility to sample the gas in the turbulence chamber even at high sampling rates without affecting the flow measurement: these gas samples may thus be analyzed by low cost industrial analyzer.

In some applications it can be useful to compensate the pressure drop in the turbulent chamber in order to use a zero point detector as pressure transducer to minimize its dimensions. In this case an active compensation system of the pressure drop can be easily realized through a ventil or an air jet inside or outside the turbulent chamber be easily realized through a ventil or an air jet inside or outside the turbulent chamber within the measuring points.

**Claims**

1. Turbulence conveyor gas flowmeter for medical use particularly for monitoring human respiratory functions comprising

a body portion (2) including a cavity having input and output openings for input and output of gasflow respectively,

a movable intercepting element (18; 29) within said cavity with turbulence chamber means (4), the movement of said intercepting element (18; 29) bringing said chamber means (4) into such position as to connect said input and output openings by a variable amount, with the gas flowing from one input to one output of said input and output openings through the chamber means (4),

transducer system means (22, 23; 26, 27; 26, 28) responsive to variable pressure drops across the input and output openings, said transducer system means (22, 23; 26, 27; 26, 28) being connected to the input and output openings by means of ducts (11)

a servo-mechanism (25) controlled by said transducer system means (22, 23; 26, 27; 26, 28) for moving said intercepting element (18; 29) and circuit means (24) providing an indication of the gasflow, said flow meter being characterized in

that said input and output openings include three openings, a first opening of which is to be connected to a patient's mouth, a second opening of which forming the input to said cavity, and a third opening of which forming the output from said cavity for conveying respectively the inspired and expired air to different paths,

that said chamber means (4) is built in the form of at least a throughpassage, said connection's variable amount being achieved by varying the degree of registration of said throughpassage with respect to said three openings,

that said intercepting element (18; 29) connects said first opening to said second opening during inspiration and to said third opening during expiration,

that the transducer system means comprises two systems (22, 23; 26, 27; 26, 28), a first one (22; 26, 27) being connected between said first opening and said second opening, and a second one (23; 26, 28) being connected between said first opening and said third opening, and

that said circuit means comprises computing means (24) connected with and receiving signals from said transducer system means (22, 23; 26, 27, 26, 28) and said servomechanism (25) for sending signals to said servo-mechanism (25) to control the operation of said servo-mechanism (25) and to provide said indication of the gas flow.

2. A turbulence conveyor gas flowmeter according to claim 1 being characterized in that said intercepting element (18; 29) is a rotary element rotated by said servo-mechanism (25) and containing two passageways which mutually exclusively connect said first opening to said second opening and said first opening to said third opening.

3. A turbulence conveyor gas flowmeter according to claim 1 or claim 2, being characterized in that said servo-mechanism (25) comprises a rotational servomotor.

4. A turbulence conveyor gas flowmeter according to one of the claims 1 through 3, being characterized in that said computing means (24) comprises a microprocessor.

5. A turbulence conveyor gas flowmeter according to claim 1, being characterized in that said movable intercepting element (18; 29) comprises a reciprocatory element.

6. A turbulence conveyor gas flowmeter according to one of the claims 1 through 5, being characterized in that breath sampling passage means are connected in the body portion (2) in communicating relationship with said throughpassage.

7. A turbulence conveyor gas flowmeter according to one of claims 1 through 6, being characterized in that said throughpassage comprises a substantially straight passage.

8. A turbulence conveyor gas flowmeter according to one of claims 1 through 6, being characterized in that said throughpassage comprises an arcuate passage.

**Patentansprüche**

1. Turbulenzförderer-Gasflußmesser für medizinische Zwecks, insebesondere zur Überwachung menschlicher Atmungsfunktionen, aufweisend

—ein einen Hohlraum beinhaltendes Gehäuseteil (2) mit Einlaß- und Auslaßöffnungen für den Eingang bzw. Ausgang des Gasflusses,

—ein im Hohlraum angeordnetes bewegliches Sperrelement (18; 29) mit einer Wirbelkammer (4), wobei die Bewegung des Sperrelementes (18; 29) die Kammer in eine derartige Lage bringt, daß die Eingangs- und Ausgangsöffnungen mit einem veränderbaren Maß angeschlossen werden, wobei das Gas von dem einen Eingang zu dem einen Ausgang der Eingangs- und Ausgangsöffnungen durch die Kammer (4) fließt,

—ein Übertragersystem (22, 23; 26, 27; 26, 28), das auf unterschiedliche Durckabfälle entlang der Eingangs- und Ausgangsöffnungen anspricht, wobei das Übertragersystem (22, 23; 26, 27; 26, 28) mit den Eingangs- und Ausgangsöffnungen durch Leitungen (11) verbunden ist,

—ein Servomechanismus (25), der durch das Übertragersystem (22, 23; 26, 27; 26, 28) gesteuert wird, um das Sperrelement (18; 29) zu bewegen, sowie ein Schaltteil (24), das eine Anzeige vorsieht,

dadurch gekennzeichnet, daß

—zu den Eingangs- und Ausgangsöffnungen drei Öffnungen gehören, wobei eine erste Öffnung mit dem Mund des Patienten verbunden werden kann, eine zweite Öffnung den Eingang des Hohlraums bildet und eine dritte Öffnung den Ausgang des Hohlraums bildet, um die eingeatmete bzw. ausgeatmete Luft über verschiedene Wege zu fördern,

—daß die Kammer (4) in Form wenigstens eines Durchganges ausgebildet ist, wobei das veränderbare Auschlußmaß dadurch erreicht wird, daß der Deckungsgrad des Durchganges bezüglich der drei Öffnungen verändert wird,

—daß das Sperrelement (18, 29) die erste Öffnung mit der zweiten Öffnung während des Einatmens und mit der dritten Öffnung während des Ausatmens verbindet,

—daß das Übertragersystem zwei Systeme (22, 23; 26, 27; 26, 28) aufweist, wobei ein erstes (22; 26, 27) zwischen die erste Öffnung und die zweite Öffnung geschaltet ist und ein zweites (23; 26, 28) zwischen die erste Öffnung und die dritte Öffnung geschaltet ist, und

—daß das Schaltteil ein Rechnerteil (24) beinhaltet, das verbunden ist mit und Signale von dem Übertragersystem (22, 23; 26, 27; 26, 28) und Servomechanismus (25) empfängt, um Signale zum Servomechanismus (25) zu senden, wodurch der Betrieb des Servomechanismus (25) gesteuert und die Anzeige des Gasflusses geschaffen werden soll.

2. Turbulenzförderer-Gasflußmesser gemäß Anspruch 1, dadurch gekennzeichnet, daß das Sperrelement (18, 29) ein drehbares Element ist, das von dem Servomechanismus (25) gedreht wird und zwei Durchgänge aufweist, welche wechselseitig ausschließlich die erste Öffnung mit der zweiten bzw. die erste Öffnung mit der dritten Öffnung verbinden.

3. Turbulenzförderer-Gasflußmesser gemäß Anspruch 1 oder 2, dadurch gekennzeichnet, daß der Servomechanismus (25) einen rotierenden Servomotor aufweist.

4. Turbulenzförderer-Gasflußmesser nach einem der Ansprüche 1—3, dadurch gekennzeichnet, daß das Rechnerteil (24) einen Mikroprozessor aufweist.

5. Turbulenzförderer-Gasflußmesser nach Anspruch 1, dadurch gekennzeichnet, daß das bewegliche Sperrelement (18, 29) ein hin- und herbewegbares Element aufweist.

6. Turbulenzförderer-Gasflußmesser nach einem der Ansprüche 1—5, dadurch gekennzeichnet, daß Atemproben-Durchgangsmittel im Gehäuseteil (2) mit dem Durchgang in Verbindung steht.

7. Turbulenzförderer-Gasflußmesser nach einem der Ansprüche 1—6, dadurch gekennzeichnet, daß der Durchgang einen im wesentlichen geradlinigen Durchgang aufweist.

8. Turbulenzförderer-Gasflußmesser nach einem der Ansprüche 1—6, dadurch gekennzeichnet, daß der Durchgang einen bogenförmigen Durchgang aufweist.

**Revendications**

1. Débitmètre de gaz à tourbillons pour usage médical, en particulier pour surveiller les fonctions respiratoires humaines, comprenant

un corps (2) présentant une cavité percée d'orifices d'entrée et de sortie permettant, respectivement, l'entrée et la sortie d'un flux gazeux,

un élément intercepteur mobile (18; 29) à l'intérieur de ladite cavité, avec un moyen (4) délimitant une chambre de turbulence, le mouvement dudit élément intercepteur (18; 29) amenant ledit moyen (4) délimitant la chambre à une position permettant d'établir, selon une valeur variable, la communication entre lesdits orifices d'entrée et de sortie, le gaz circulant dans le moyen (4) délimitant la chambre, de l'une des entrées à l'une des sorties desdits orifices d'entrée et de sortie,

des moyens (22, 23; 26, 27; 26, 28) formant un système transducteur et réagissant à des chutes de pression variables à travers les orifices d'entrée et de sortie, lesdits moyens (22, 23; 26, 27; 26, 28) formant un système transducteur étant reliés aux orifices d'entrée et de sortie au moyen de canaux (11),

un servomécanisme (25), commandé par lesdits moyens (22, 23; 26, 27; 26, 28) formant un systéme transducteur, afin d'imprimer un mouvement audit élément intercepteur (18; 29), ainsi qu'un moyen de circuit (24) fournissant une indication relative au débit gazeux, ledit débitmètre étant caractérisé par le fait

que lesdits orifices d'entrée et de sortie comprennent trois orifices, parmi lesquels un premier orifice est destiné à être relié à la bouche d'un patient, un deuxième orifice forme l'entrée dans ladite cavité et un troisième orifice forme la sortie de ladite cavité, pour acheminer vers différents trajets l'air respectivement inspiré et expiré, que ledit moyen (4) délimitant la chambre est réalisé sous la forme d'au moins un passage de circulation traversante, ladite valeur variable de la communication étant obtenue en faisant varier le degré d'alignement dudit passage de circulation traversante par rapport auxdits trois orifices, que ledit élément intercepteur (18; 29) raccorde ledit premier orifice audit deuxième orifice lors de l'inspiration, et audit troisième orifice lors de l'expiration,

que les moyens formant un système transducteur comprennent deux systèmes (22, 23; 26, 27; 26, 28), dont un premier (22; 26, 27) est intercalé entre ledit premier orifice et ledit deuxième orifice, et un second (23; 26, 28) est intercalé entre ledit premier orifice et ledit troisième orifice, et

que ledit moyen de circuit consiste en un moyen de calcul (24) raccordé auxdits moyens (22, 23; 26, 27, 26, 28) formant un système transducteur et audit servomécanisme (25), dont il reçoit les signaux, pour délivrer des signaux audit servomécanisme (25) afin de commander le fonctionnement dudit servomécanisme (25) et de fournir ladite indication relative au débit gazeux.

2. Débitmètre de gaz à tourbillons selon la revendication 1, caractérisé par le fait que ledit élément intercepteur (18; 29) est un élément rotatif mis en rotation par ledit servomécanisme (25), et renfermant deux passages qui font exclusivement communiquer l'un avec l'autre ledit premier orifice et ledit deuxième orifice, et ledit premier orifice et ledit troisième orifice.

3. Débitmètre de gaz à tourbillons selon la revendication 1 ou la revendication 2, caractérisé par le fait que ledit servomécanisme (25) consiste en un servomoteur rotatif.

4. Débitmètre de gaz à tourbillons selon l'une des revendications 1 à 3, caractérisé par le fait que ledit moyen de calcul (24) consiste en un microprocesseur.

5. Débitmètre de gaz à tourbillons selon la revendication 1, caractérisé par le fait que ledit élément intercepteur mobile (18; 29) consiste en un élément animé d'un vaet-vient.

6. Débitmètre de gaz à tourbillons selon l'une des revendications 1 à 5, caractérisé par le fait que des passages d'échantillonage respiratoire sont reliés dans le corps (2), en communication avec ledit passage de circulation traversante.

7. Débitmètre de gaz à tourbillons selon l'une des revendications 1 à 6, caractérisé par le fait que ledit passage de circulation traversante consiste en un passage sensiblement rectiligne.

8. Débitmètre de gaz à tourbillons selon l'une des revendications 1 à 6, caractérisé par le fait que ledit passage de circulation traversante consiste en un passage curviligne.

FIG. 1

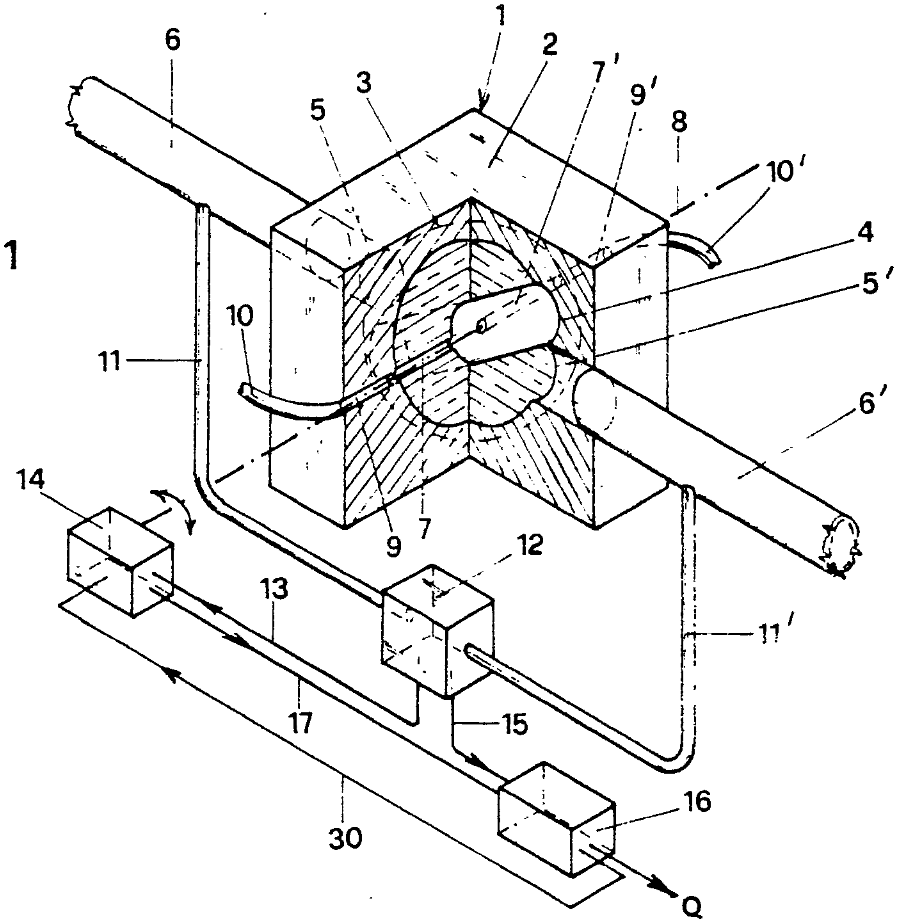
1
2
3
4
5
5'
6
6'
7
7'
8
9
9'
10
10'
11
11'
12
13
14
15
16
17
30
Q

FIG. 2

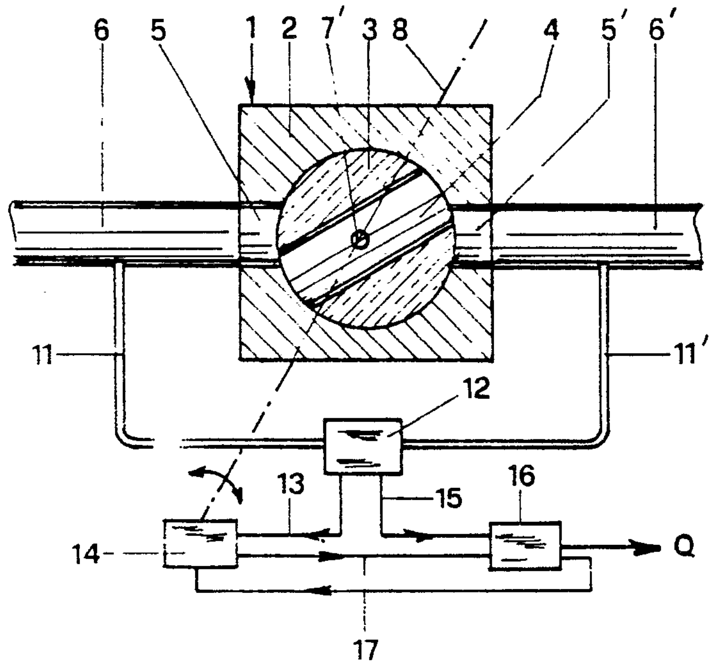
6
5
1
2
7'
3
8
4
5'
6'
11
11'
12
13
15
16
14
Q
17

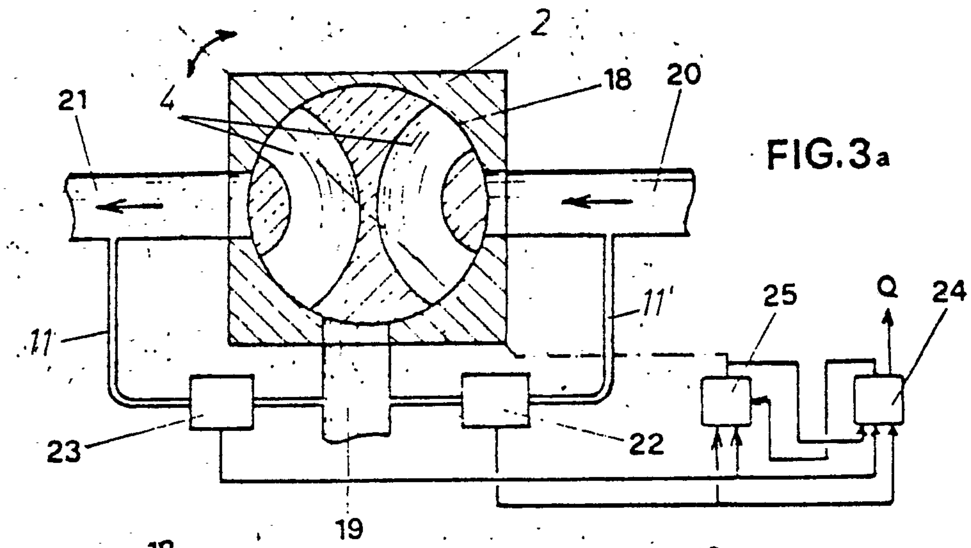


FIG.3a

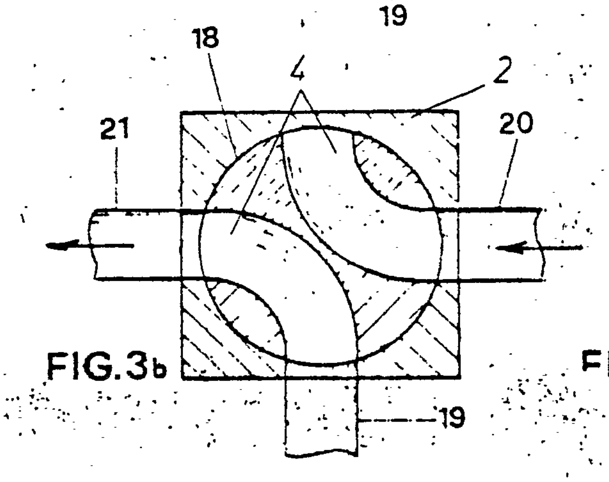


FIG.3b

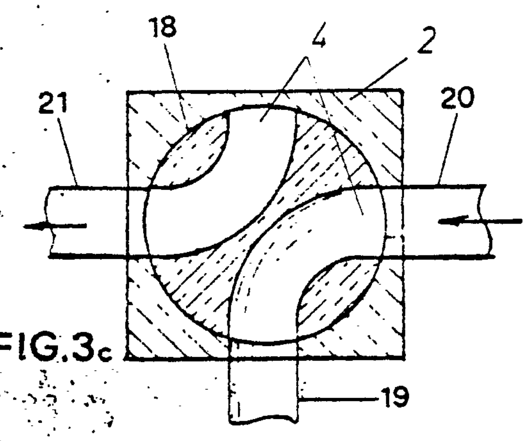


FIG.3c

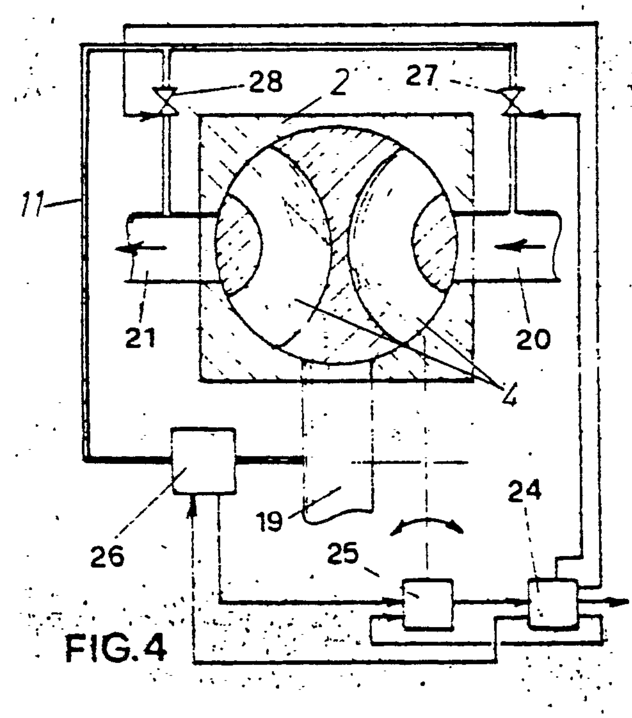


FIG.4

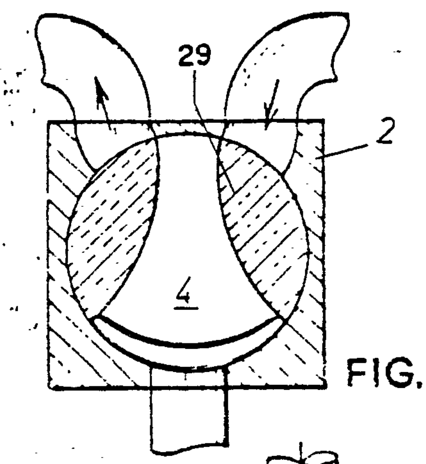


FIG.5

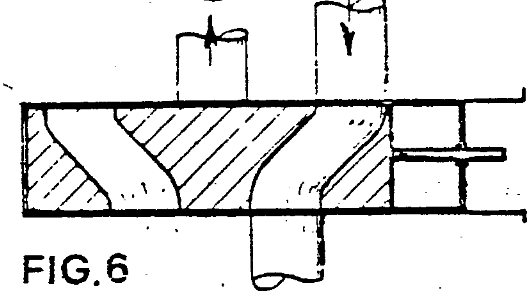

FIG.6